# EUROPEAN PATENT APPLICATION

(11) **EP 3 162 334 A1**
(43) Date of publication of application: **03.05.2017**
(21) Application number: 15814087.1
(22) Date of filing: 12.05.2015
(51) Int. Cl.: A61F 13/15, A61F 13/472, A61F 13/511, A61F 13/53, A61F 13/539

(54) **METHOD FOR MANUFACTURING ABSORBENT ARTICLE**

(30) Priority: 30.06.2014 JP 2014135192
(71) Applicant: Unicharm Corporation, Ehime 799-0111 (JP)
(72) Inventor: TANIO, Toshiyuki, Kanonji-shi Kagawa 769-1602 (JP); KUDO, Jun, Kanonji-shi Kagawa 769-1602 (JP); TAKAHASHI, Yuji, Kanonji-shi Kagawa 769-1602 (JP); SHIMA, Asami, Kanonji-shi Kagawa 769-1602 (JP); FUJITA, Tomoyuki, Kanonji-shi Kagawa 769-1602 (JP)
(74) Representative: Peter, Julian
(86) International application number: PCT/JP2015/063655
(87) International publication number: WO 2016/002349

(57) **Abstract**

The purpose of the present disclosure is to provide a method for manufacturing an absorbent article, the method allowing the application, at a desired position and at a high rate, of water-disintegrable microcapsules containing a volatile functional component. The manufacturing method according to the present disclosure has the following constitution. A method for manufacturing an absorbent article (1) including a liquid permeable layer (2), a liquid impermeable layer (3), and an absorbent layer (4), said method comprising: a step for forming a functional composition by mixing water-disintegrable microcapsules containing a volatile functional component with a solvent capable of keeping the functional component as contained in the microcapsules; a step for coating the functional composition on at least a portion of a surface of a layer to be coated to form a functional composition-coated region (8) on the surface to be coated; and a step for stacking the liquid permeable layer (2), and the absorbent layer (4) and the liquid impermeable layer (3) with an adhesive-coated region (9) coated with an adhesive interposed therebetween.

## Description

### Technical Field

The present disclosure relates to method of producing an absorbent article.

### Background Art

Absorbent articles having an aromatic function and a cooling function are known. For example, PTL 1 describes an absorbent article comprising a cooling material that includes a refrigerant, between a front sheet and a back sheet. In the absorbent article of Reference 1, the refrigerant is protected in water-soluble polymer cells, the refrigerant being released after having contacted with body fluid or the like from the user. Reference 1 also describes production of an absorbent article by packing a cooling material, that includes a refrigerant protected in polymer cells, into the pores of a surface treatment roll, and transferring it onto a hot-melt adhesive-coated material.

### Citation List

### Patent Literature

PTL 1 Japanese Unexamined Patent Publication No. 2010-234031

### Summary of Invention

### Technical Problem

In the method of producing an absorbent article described in PTL 1, the cooling material that includes a refrigerant protected in polymer cells is coated in its original powder form, and therefore it is in need of improvement as the cooling material tends to fly out into the air, and rapid production is difficult.

It is therefore an object of the present disclosure to provide a method of producing an absorbent article that allows water-disintegratable microcapsules encapsulating a volatile functional component to be rapidly coated onto desired locations.

### Solution to Problem

The present inventors have devised a method of producing an absorbent article including a liquid-permeable layer, a liquid-impermeable layer and an absorbing layer between the liquid-permeable layer and liquid-impermeable layer, including the steps of: forming a functional composition by mixing water-disintegratable microcapsules encapsulating a volatile functional component and a solvent capable of holding the functional component encapsulated in the microcapsules; forming a functional composition-coated region on a coating surface of a layer to be coated by coating the functional composition onto at least a portion of the coating surface of the layer to be coated; and stacking the liquid-permeable layer, the absorbing layer and the liquid-impermeable layer sandwiching an adhesive-coated region that is coated with an adhesive.

### Advantageous Effects of Invention

The method of producing an absorbent article according to the disclosure allows water-disintegratable microcapsules encapsulating a volatile functional component to be rapidly coated onto desired locations.

### Brief Description of Drawings

Fig. 1 is a plan view of an absorbent article produced by a method according to an embodiment of the disclosure.
Fig. 2 is a cross-sectional view along cross-section II-II of Fig. 1.
Fig. 3 is a diagram illustrating a method of producing an absorbent article according to an embodiment of the disclosure.
Fig. 4 is a diagram illustrating a method of producing an absorbent article according to an embodiment of the disclosure.
Fig. 5 is a plan view of an absorbent article produced by a method according to another embodiment of the disclosure.

### Description of Embodiments

### [Definitions]

The definitions of several of the terms used as used herein will now be explained.

### [Microcapsules]

As used herein, "microcapsules" means capsules having sizes with diameters of 1 to 1,000 µm, and having spaces that encapsulate a core material (functional component).

The outer shapes of the capsules are not particularly restricted so long as they allow the core material to be encapsulated and release of the core material to be controlled, and for example, the outer shapes may be spherical, amorphous or the like. The capsules may be mononuclear having a single space for holding the core material, or polynuclear having multiple spaces for holding the core material. The shapes of the spaces may be spherical, amorphous or other spatial forms.

Examples of microcapsules are ones having a spherical outer appearance and polynuclear spaces.

### [Water disintegratability]

As used herein, "water disintegratability" is a term relating to microcapsules and refers to the property of disintegrating to an extent that releases the encapsulated functional component upon contact with a fluid (aqueous solution), such as body fluid. Specifically, this includes cases where the material of the microcapsules dissolves in water and disintegrates when the microcapsules contact with fluid, and cases where, when the microcapsules contact with fluid, the microcapsules become imbibed with water and lose their strength, resulting in destruction of the microcapsules and disintegration.

### [Wearer]

As used herein, "wearer" means the wearer of the absorbent article, and for example, the wearer may be an infant or care receiver in the case of a disposable diaper, or a female in the case of a sanitary napkin.

### [User]

As used herein, "user" means the user of the absorbent article, and in addition to the wearer mentioned above, it includes any person that fits the absorbent article onto the wearer, such as a mother or caregiver in the case of disposable diaper, for example.

### [Facing sides]

As used herein, "facing sides" means the sides of two adjacent layers that are mutually facing.

The method of producing an absorbent article of the disclosure, and the absorbent article produced by the method, will now be explained in detail with reference to the accompanying drawings as necessary (the method of producing an absorbent article will also be referred to hereunder as "production method").

For clarity of the disclosure, an absorbent article produced by the production method according to an embodiment of the disclosure will be described first.

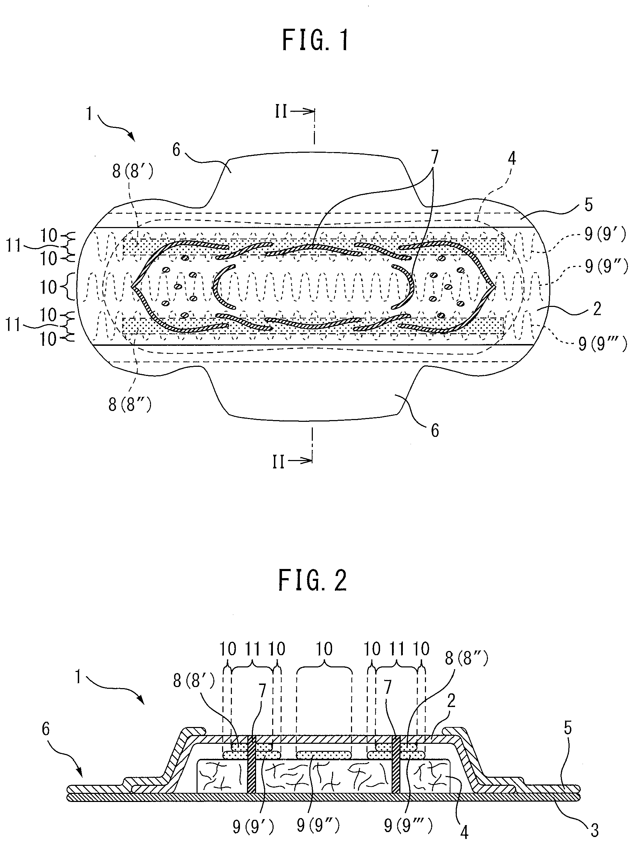

Fig. 1 is a front view of an absorbent article, and more specifically a front view of a sanitary napkin, produced by the production method according to an embodiment of the disclosure. Fig. 2 is a cross-sectional view along cross-section II-II of Fig. 1.

The absorbent article 1 shown in Fig. 1 and Fig. 2 has a top sheet 2 as a liquid-permeable layer, a back sheet 3 as a liquid-impermeable layer, and an absorbent body 4 as an absorbing layer, between the top sheet 2 and back sheet 3.

It should be noted that Fig. 2 is magnified in the thickness direction of the absorbent article 1, for greater clarity of the disclosure. For example, for representation of the functional composition-coated regions 8 (8' and 8") in Fig. 2, a space is shown between the adhesive-coated region 9" and the top sheet 2, but in actuality the adhesive-coated region 9" has the absorbent body 4 and the top sheet 2 adhered together.

The absorbent article 1 shown in Fig. 1 comprises a pair of side flaps 6 on both edges in the lengthwise direction of the absorbent article 1, formed from side sheets 5 and a back sheet 3, to anchor the absorbent article 1 to the clothing of the wearer, such as shorts.

The absorbent article 1 shown in Fig. 1 also has three adhesive-coated regions 9 (9', 9", 9"') coated with an adhesive and extending in the planar direction of the absorbent article 1, between the top sheet 2 and absorbent body 4. The three adhesive-coated regions 9 (9', 9", 9"') are disposed along the lengthwise direction of the absorbent article 1, at fixed intervals in the widthwise direction of the absorbent article 1. In each adhesive-coated region 9, the adhesive is disposed along the lengthwise direction of the absorbent article 1 while reciprocating (in a Z-shape) in the widthwise direction of the absorbent article 1. The three adhesive-coated regions 9 (9', 9", 9"') are also disposed in contact with the top sheet 2.

The absorbent article 1 shown in Fig. 1 also has two functional composition-coated regions 8 (8', 8") coated with a functional composition and extending in the planar direction of the absorbent article 1, between the top sheet 2 and absorbent body 4. The functional composition-coated regions 8' and 8" are each disposed in a sheet-like manner between the top sheet 2 and absorbent body 4, in contact with the adhesive-coated regions 9' and 9"'. The functional composition-coated regions 8' and 8" are also disposed in contact with the top sheet 2. The composition of the functional composition is described below.

The adhesive-coated region 9" has a adhered region 10 where it is directly adhered with the top sheet 2 and absorbent body 4. Each of the adhesive-coated regions 9' and 9'" also has a adhered region 10 where they are directly adhered with the top sheet 2 and absorbent body 4, and a microcapsule-holding region 11.

The adhered regions 10 are not adhered with the functional composition-coated regions 8, and more specifically, the adhered regions 10 adhere the top sheet 2 and absorbent body 4 without including a solvent as a component of the functional composition.

In the microcapsule-holding regions 11, the adhesive having a pressure-sensitive adhesive property holds microcapsules forming the functional composition.

The absorbent article 1 shown in Fig. 1 has a plurality of embossed sections 7 formed by embossing the top sheet 2 and the absorbent body 4 sandwiching the functional composition-coated regions 8. The embossed sections 7 hold the functional component encapsulated in the microcapsules in the areas overlapping with the functional composition-coated regions 8 in the thickness direction of the absorbent article 1.

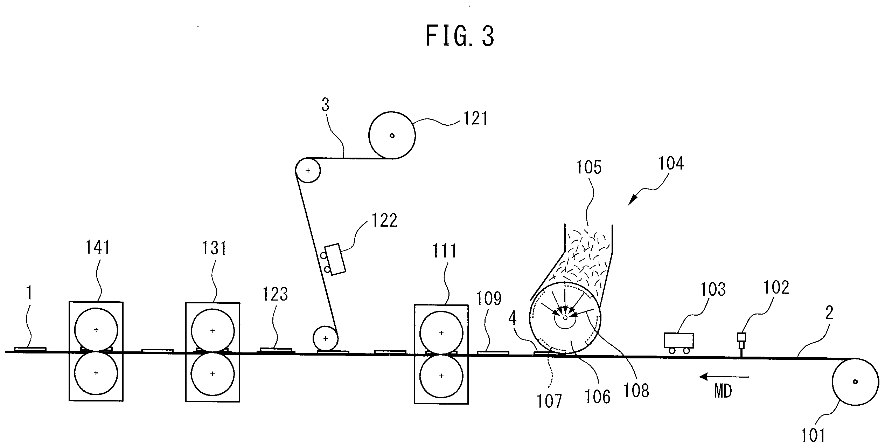

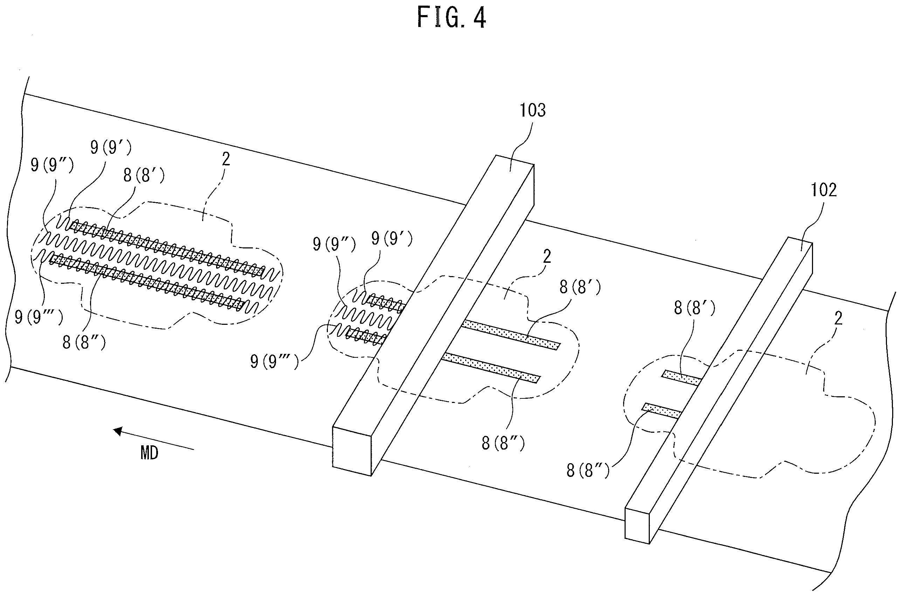

The production method of the disclosure will now be described with reference to the embodiment thereof illustrated in Fig. 3 and Fig. 4, as necessary.

Fig. 3 is a diagram for illustration of a method of producing an absorbent article according to an embodiment of the disclosure, and specifically for illustration of the method of producing an absorbent article shown in Fig. 1 and Fig. 2. Fig. 4 is a magnified perspective view of the process of coating the top sheet 2 with a functional composition using a functional composition-coating device 102.

The absorbent article produced by the production method of the disclosure includes a liquid-permeable layer, a liquid-impermeable layer, and an absorbing layer between the liquid-permeable layer and the liquid-impermeable layer. The liquid-permeable layer, liquid-impermeable layer and absorbing layer are referred to respectively as the top sheet, back sheet and absorbent body in the technical field.

The liquid-permeable layer, liquid-impermeable layer and absorbing layer may be formed of materials known in the technical field, but the liquid-impermeable layer is preferably a nonwoven fabric from the viewpoint of holding the solvent composing the functional composition.

The production method of the disclosure includes a step of forming a functional composition by mixing water-disintegratable microcapsules that encapsulate a volatile functional component, and a solvent capable of holding the functional component encapsulated in the microcapsules, (also referred to as "functional composition-forming step"). The functional composition will be described in detail below.

The production method of the disclosure includes a step of forming a functional composition-coated region on the coating surface of a layer to be coated by coating the functional composition onto at least a portion of the coating surface of the layer to be coated, (also referred to as "functional composition-coated region-forming step").

The layer to be coated may be the liquid-permeable layer or absorbing layer, and the coating surface may be the non-skin contact surface of the liquid-permeable layer or the liquid-permeable layer side surface of the absorbing layer.

When the coating surface is the non-skin contact surface of the liquid-permeable layer, absorbed fluid can rapidly reach the microcapsules, allowing the functional component to be rapidly released.

As used herein, coating of the layer to be coated and the coating surface should be coated with the functional composition.

Also, when the absorbent article includes an auxiliary sheet layer between the liquid-permeable layer and the absorbing layer, the coating surface of the layer to be coated will include the liquid-permeable layer side surface of the auxiliary sheet and the absorbent body side surface of the auxiliary sheet.

In the production method of the disclosure, the functional composition can be coated using a coating machine known in the technical field, such as a roll-type coating machine, curtain-type coating machine, slit-type coating machine, spray-type coating machine, dip-type coating machine, bead-type coating machine, flexo-type coating machine or gravure-type coating machine.

The functional composition is preferably coated with a contact-type coating machine having the exit nozzle for the functional composition in contact with the coating surface. This is to allow the functional composition to be situated at the desired positions, i.e. to allow the functional composition-coated regions to be situated at the desired positions, without scattering of the functional composition.

Contact-type coating machines include roll-type coating machines, slit-type coating machines, dip-type coating machines, bead-type coating machines, flexo-type coating machines and gravure-type coating machines.

In the embodiment illustrated in Fig. 1 and Fig. 2, the functional composition-coated regions 8 are formed in a sheet-like manner in contact with the top sheet 2, but the functional composition-coated regions for the production method of the disclosure are not limited to those shown in Fig. 1 and Fig. 2 and may be in a sheet-like form, linear form, spiral-form, Z-shape, linear form or dotted form. The functional composition-coated region may be formed along the lengthwise direction or widthwise direction of the absorbent article. The functional composition-coated region may also be formed at the center in the lengthwise direction of the absorbent article, and formed at both edges in the lengthwise direction of the absorbent article.

In the production method of the disclosure, the functional composition is coated so that the basis weight of the functional composition in the functional composition-coated region is preferably 1 to 12 g/m² and more preferably 2 to 10 g/m². If the basis weight is lower than 1 g/m², the functional component may be less able to exhibit its function, and if the basis weight is higher than 12 g/m², the solvent may inhibit adhering by the adhesive.

The production method of the disclosure includes a step of stacking the liquid-permeable layer, absorbing layer and liquid-impermeable layer sandwiching the adhesive-coated region that has been coated with an adhesive (hereunder also referred to as "stacking step").

The adhesive-coated region can be formed at any desired locations between the liquid-permeable layer and the absorbing layer and between the absorbing layer and the liquid-impermeable layer.

Likewise, when the absorbent article includes an auxiliary sheet layer between the liquid-permeable layer and the absorbing layer, the adhesive-coated region can be formed at any desired locations between the liquid-permeable layer and the auxiliary sheet layer and between the auxiliary sheet layer and the absorbing layer.

In the production method of the disclosure, the adhesive may be coated using an apparatus known in the technical field, such as a hot-melt gun. Using a hot-melt gun, for example, the adhesive is coated along the lengthwise direction of the absorbent article while reciprocating in the widthwise direction of the absorbent article, forming adhesive-coated regions 9 as shown in Fig. 1.

In the production method of the disclosure, the adhesive forming the adhesive-coated region may be an adhesive that is known in the technical field, such as a hot-melt adhesive.

The shapes of the adhesive-coated region formed in the production method of the disclosure is not particularly restricted and may be spiral forms, Z-shapes, linear forms or dotted forms, for example, and the adhesive-coated region that is formed may be situated along the lengthwise direction or widthwise direction of the absorbent article.

The layer to be coated has a fiber density of preferably 0.02 to 0.1 g/cm³ and more preferably 0.04 to 0.08 g/cm³. If the layer to be coated has such a fiber density, the coated functional composition will tend to be easily incorporated into the layer to be coated, and the coated functional composition will be less likely to remain on the surface of the layer to be coated, thus helping to prevent fly-off of the functional composition and especially the microcapsules during transport of the layer to be coated.

The layer to be coated has a thickness of preferably 0.2 to 2.0 mm and more preferably 0.4 to 1.0 mm. If the thickness is within this range, the functional composition will be less likely to directly contact the skin of the wearer. The functional composition will also act more readily on the skin of the wearer.

The thickness of the layer to be coated is measured using an FS-60DS by Daiei Kagaku Seiki Mfg. Co., Ltd. (probe: 15 cm², measuring load: 3 gf/cm²). Specifically, the thickness of the layer to be coated is measured at 5 locations with an FS-60DS after standing for 24 hours in a thermostatic chamber at 20°C, and the mean value for the thickness is used.

As used herein, the fiber density is that calculated by dividing the basis weight of the layer to be coated by its thickness.

The basis weight is calculated by dividing the mass of the layer to be coated by its area, after standing for 24 hours in a thermostatic chamber at 20°C.

The thickness (mm) is measured in the manner described above.

In the production method of the disclosure, the coating surface (or a surface facing the coating surface) has an adhesive-coated region, and in the functional composition-coated region-forming step, "at least a portion" of the adhesive-coated region of the coating surface (or the region facing the adhesive-coated region of the coating surface) are coated with a functional composition, the adhesive-coated region preferably having microcapsule-holding region that hold microcapsules, in the areas where they overlap with the functional composition-coated region in the thickness direction of the absorbent article.

With this construction, since the microcapsule-holding region (for example, the region indicated by reference numeral 11 in Fig. 1 and Fig. 2) hold the microcapsules, the positions of the first functional component will be less likely to shift during use.

When microcapsule-holding region is present on the non-skin contact surface of the liquid-permeable layer, fluid that has passed through the liquid-permeable layer will be able to reach the microcapsules more rapidly, thereby allowing the first functional component to be released with a smaller amount of fluid.

In the production method of the disclosure, the coating surface (or a surface facing the coating surface) has adhesive-coated region, and in the functional composition-coated region-forming step, "portions" of the adhesive-coated region of the coating surface (or the region facing the adhesive-coated region of the coating surface) are coated with a functional composition, the adhesive-coated region preferably having a microcapsule-holding region that hold microcapsules, in the areas where they overlap with the functional composition-coated region in the thickness direction of the absorbent article, and preferably having an adhered region where the liquid-permeable layer and the absorbing layer are directly or indirectly adhered, in the areas overlapping with the functional composition-coated region in the thickness direction of the absorbent article.

If the absorbent article has an adhered region, the absorbent article will be resistant to twisting during use.

The adhered region also preferably does not include the solvent composing the functional composition. This is because the solvent tends to inhibit direct or indirect adhering of the liquid-permeable layer and the absorbing layer by the adhesive.

As used herein, "the adhered region does not include the solvent composing the functional composition" means that the adhesive composing the adhered region includes the solvent in an amount of 0 to 5 mass%.

The production method of the disclosure may include, after the functional composition-coated region-forming step, a step of coating an adhesive onto at least a portion of the functional composition-coated region (or the region facing the functional composition-coated region), forming adhesive-coated region that overlap with the functional composition-coated region in the thickness direction of the absorbent article.

The step of forming the functional composition-coated region and the step of forming the adhesive-coated region may be carried out in any order.

The production method of the disclosure may further include, after the functional composition-coated region-forming step, a step of forming an embossed section by embossing at least the liquid-permeable layer and absorbing layer in a manner sandwiching the functional composition-coated region, as shown in Fig. 1 and Fig. 2. The solvent composing the functional composition in the functional composition-coated region tends to inhibit direct or indirect adhering between the liquid-permeable layer and the absorbing layer by the adhesive. If the absorbent article has an embossed section, then adhering between the liquid-permeable layer and the absorbing layer will be stronger, and the absorbent article will be less likely to twist during use.

Since the embossed section holds the first functional component encapsulated in the microcapsules, the embossed section can continue to hold the microcapsules, and thus the first functional component, in the prescribed position. In addition, since the embossed section has high fiber density, the fluid will tend to be preferentially guided toward the embossed section, thereby allowing the first functional component to be released with a small amount of fluid.

When the absorbent article includes an auxiliary sheet layer between the liquid-permeable layer and the absorbing layer, the surface on the liquid-permeable layer side or the surface on the absorbing layer side of the auxiliary sheet layer may be coated with a functional composition in the functional composition-coated region-forming step, to form functional composition-coated region on the surface on the liquid-permeable layer side or the surface on the absorbing layer side.

The embodiment shown in Fig. 3 and Fig. 4 will now be explained.

A functional composition is coated from a functional composition-coating device 102 onto the surface of a belt-shaped top sheet 2 on which the absorbent body is to be stacked (the non-skin contact surface), which has been wound out from a top sheet roll 101, thus forming functional composition-coated regions 8 (8' and 8") on the top sheet 2. An adhesive is then coated from an adhesive coater 103 onto the top sheet 2 forming three adhesive-coated regions 9 (9', 9" and 9"') sandwiching the functional composition-coated regions 8 (8' and 8").

Next, the absorbent body 4 discharged from an absorbent body production apparatus 104 is stacked onto the top sheet 2 having the functional composition-coated regions 8 and adhesive-coated regions 9, to form a stacked body 109. The absorbent body production apparatus 104 is one that is known in the technical field, the absorbent body production apparatus 104 shown in Fig. 3 having a material feeder 105, a suction drum 106, a concave mold 107 formed on the outer peripheral surface of the suction drum 106, and a suction section 108.

The stacked body 109 is then embossed with a pair of embossing rolls 111 to form embossed sections 7 on the stacked body 109.

A belt-shaped back sheet 3 that has been wound out from a back sheet roll 121 is coated with an adhesive from an adhesive coater 122, and stacked onto the stacked body 109 on which the embossed sections 7 have been formed, to form a stacked body 123. A pair of embossing rolls 131 are then used for round embossing of the stacked body 123 into the shape of an absorbent article, which is cut into the shape of an absorbent article with a cutter 141, to complete the absorbent article 1.

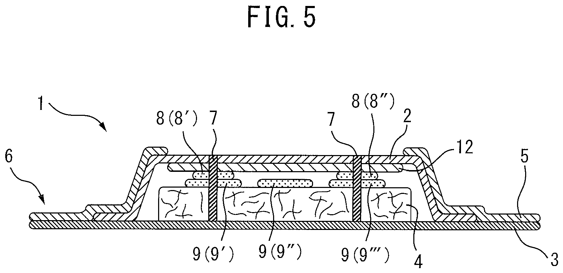

Fig. 5 is a cross-sectional view of an absorbent article produced by the production method according to another embodiment of the disclosure. Fig. 5 corresponds to cross-section II-II of Fig. 1. The absorbent article 1 shown in Fig. 5 includes an auxiliary sheet 12 between the top sheet 2 and the absorbent body 4, the functional composition-coated regions 8 (8' and 8") and adhesive-coated regions 9 (9', 9" and 9"') being formed between the auxiliary sheet 12 and the absorbent body 4. In the absorbent article 1 shown in Fig. 5, the functional composition-coated regions 8 are disposed in contact with the auxiliary sheet 12, and the adhesive-coated regions 9 are disposed in contact with the absorbent body 4. Explanation of the other sections will be omitted since they are similar to the embodiments shown in Fig. 1 and Fig. 2.

The absorbent article 1 shown in Fig. 5 is produced by carrying out the same processes as in Fig. 3, except that in Fig. 3, an auxiliary sheet (not shown) wound out from an auxiliary sheet roll (not shown) is stacked on the top sheet 2 that has been wound out from the top sheet roll 101, optionally sandwiching an adhesive, and the auxiliary sheet is coated with a functional composition from the functional composition-coating device 102, after which it is coated with an adhesive from the adhesive coater 103.

In the embodiment shown in Fig. 5, an auxiliary sheet layer is added between the functional composition and the wearer, and therefore the function of the functional component on the skin of the wearer tends to be more subdued.

The material of the auxiliary sheet may be the same as the top sheet, such as a nonwoven fabric.

In the embodiment shown in Fig. 5, both the functional composition-coated region and the adhesive-coated region are disposed between the auxiliary sheet layer and the absorbing layer, but in the production method according to a different embodiment of the disclosure, the functional composition-coated region and/or the adhesive-coated region are formed between the liquid-permeable layer and an auxiliary sheet layer. This will facilitate dissolution of the microcapsules by absorbed fluid, so that the first functional component will be more easily released.

The functional composition includes water-disintegratable microcapsules that encapsulate a volatile functional component, and a solvent capable of holding the functional component encapsulated in the microcapsules.

The functional composition may also include a volatile second functional component dissolved in the solvent.

As used herein, the functional component encapsulated in the microcapsules may be referred to as the "first functional component", to distinguish it from the second functional component.

Also as used herein, the first functional component and/or second functional component may be collectively referred to simply as "functional components".

The second functional component may be coated onto the coating surface of the layer to be coated, as a functional composition containing microcapsules, the second functional component and a solvent. Alternatively, the second functional component may be coated onto the coating surface of the layer to be coated after having been dissolved in a solvent if necessary, separately from the functional composition containing the microcapsules and solvent.

The respective functions of the first functional component and second functional component are not particularly restricted so long as they are functions that provide comfort for the user compared to when those components are not present, and examples include functions selected from the group consisting of aromatic functions, cooling functions, deodorant functions, antibacterial functions, skin care functions, and any combination thereof.

Functional components with an aromatic function are not particularly restricted so long as they are used as aromatics in the technical field, and examples include highly volatile aromatics with boiling points of no higher than about 250°C, and moderately volatile aromatics with boiling points of about 250°C to about 300°C.

Functional components with aromatic functions may collectively be referred to as "aromatic components".

Examples of highly volatile aromatics include anisole, benzaldehyde, benzyl acetate, benzyl alcohol, benzyl formate, isobornyl acetate, citronellal, citronellol, citronellyl acetate, paracymene, decanal, dihydrolinalool, dihydromyrcenol, dimethylphenylcarbinol, eucalyptol, I-carvone, geranial, geraniol, geranyl acetate, geranylnitrile, nerol, neryl acetate, nonyl acetate, linalool, linalyl acetate, phenylethyl alcohol, α-pinene, β-pinene, γ-pinene, α-ionone, β-ionone, γ-ionone, α-terpineol, β-terpineol, terpinyl acetate and tentarome.

Examples of moderately volatile aromatics include amylcinnamaldehyde, methyl dihydrojasmonate, isoamyl salicylate, β-caryophyllene, cedrene, cedryl methyl ether, cinnamic alcohol, coumarin, dimethylbenzyl carbinyl acetate, ethylvanillin, eugenol, isoeugenol, γ-methylionone, heliotropin, hexyl salicylate, cis-3-hexenyl salicylate, phenylhexanol, vanillin and pentalide.

These aromatic components contain aromatics with green herbal fragrances. Aromatics with green herbal fragrances can safely and conveniently alleviate concomitant symptoms of menstrual discharge, and particularly symptoms creating psychological discomfort, without producing physical stimulation to the body and without oral administration, and they provide a comfortable feel for the user.

The aforementioned green herbal fragrances are incense tones including green fragrances (green notes) or herbal fragrances (herbal notes). Green fragrances are the refreshing incense tones of grass or young leaves. Herbal fragrances (herbal notes) are incense tones characteristically having natural, medicinal fragrances obtained using herbs.

Examples of aromatics having green herbal fragrances include cis-3-hexenol, cis-3-hexenyl formate, cis-3-hexenyl acetate, cis-3-hexenyl propionate, cis-3-hexenyl butyrate, trans-2-hexenal, trans-2-hexenyl acetate, hexyl acetate, styrallyl acetate, 2-methyl-3-(3,4-methylenedioxyphenyl)-propanal (Helional, by IFF), 3(4)-(5-ethylbicyclo[2,2,1]heptyl-2)-cyclohexanol, 2-pentyloxyallyl glycolate (Allylamyl glycolate, by IFF), 4-methyl-3-decen-5-ol (Undecavertol, by Givaudan), hexylaldehyde, 2,4-dimethyl-3-cyclohexenylcarboxyaldehyde (Tripral, by IFF) and phenylacetaldehyde.

Aromatics having green herbal fragrances include I-menthol, 1,8-cineol, methyl salicylate, citronellal, camphor, borneol, isobornyl acetate, terpinyl acetate, eugenol, anethole, 4-methoxybenzyl alcohol and estragole.

Functional components having the cooling functions (functional components having cooling functions will also be referred to collectively as "cooling components") include those known as cooling materials in the technical field, and examples include components that act on receptor activated channels in skin nerves (TRPM8), such as menthol (for example, I-menthol) and its derivatives, methyl salicylate, camphor, and essential oils derived from plants (such as mint or eucalyptus). In addition, functional components having such a cooling function include components that lower the surrounding temperature by heat of vaporization, including alcohols, such as methanol and ethanol.

Functional components having the deodorant functions (functional components with deodorant functions will also be referred to collectively as "deodorant components") include those known as deodorants in the technical field.

Examples of the skin care functions include anti-inflammatory functions, antipruritic functions, eruption-preventing functions and moisturizing functions, and examples of functional components with the skin care functions include menthol and methyl salicylate.

Functional components with the skin care functions may also be referred to collectively as skin care components, and functional components having anti-inflammatory functions, antipruritic functions, eruption-preventing functions and moisturizing functions may be referred to collectively as anti-inflammatory components, antipruritic components, eruption-preventing components and moisturizing components, respectively.

The first functional component and the second functional component both have a volatile property.

The volatile property is one with a preferred vapor pressure that will differ depending on the function to be exhibited by the functional component, and for example, the first functional component and second functional component have a vapor pressure of preferably 30 Pa or greater, more preferably 50 Pa or greater and even more preferably 70 Pa or greater at 25°C, 1 atmosphere. If the vapor pressure is too low, it will tend to be difficult to exhibit the desired function, and if the vapor pressure is too high, the functional component may volatilize off before the user uses the absorbent article, sometimes resulting in its amount being reduced or its function being over-exhibited on the skin of the wearer.

The water-disintegratable microcapsules are components that hold the first functional component on the inner side, and upon contacting with fluid, such as body fluid, they dissolve and release the first functional component to the exterior. The released first functional component gasifies by body heat of the wearer, exhibiting its function to the skin of the wearer.

The degree of water disintegratability of the microcapsules will differ depending on how the first functional component is to be released after contacting with the fluid, and for example, higher water disintegratability is preferred for rapid release of the first functional component after contacting with fluid, while lower water disintegratability is preferred for sustained release of the first functional component.

In the production method of the disclosure, when the microcapsules disintegrate by dissolution in water, the microcapsules have a water solubility in the range of preferably 10 to 300 g, more preferably 20 to 200 g and even more preferably 30 to 100 g, in 100 g of water at 25°C.

The water solubility is measured according to the flask method of OECD Guidelines No. 105, except that the testing temperature is set at 25°C.

The microcapsules are preferably insoluble in the solvent used as the dispersing medium for the microcapsules, and preferably they do not swell in the solvent used as the dispersing medium for the microcapsules. This is from the viewpoint of protecting the first functional component encapsulated in them.

For example, the microcapsules may have a solvent solubility of preferably no greater than 1.0 g, more preferably no greater than 0.5 g and even more preferably no greater than 0.1 g, in 100 g of solvent as the dispersing medium of the microcapsules, at 25°C.

The solvent solubility is evaluated by adding 1.0 g (or, 0.5 g, 0.1 g) of sample to 100 g of solvent at 25°C, allowing the mixture to stand for 24 hours and gently stirring if necessary, and then visually evaluating whether or not the sample has dissolved.

The material for such microcapsules may be, for example, a saccharide, such as a monosaccharide (for example, glucose), disaccharide (for example, sucrose), polysaccharide (for example, dextrin, glucomannan, sodium alginate or water-soluble starch), gelatin, a water-soluble polymer (for example, polyvinyl alcohol or polyvinyl acetate), or the like.

The microcapsules include microcapsules that pass through a sieve with a mesh opening of 75 µm and remain on a sieve with a mesh opening of 45 µm, at preferably 50 mass% or greater and more preferably 70 mass% or greater. This is from the viewpoint of the dispersibility of the microcapsules in the solvent, and coatability.

Such microcapsules are commercially available, such as INCAP™ marketed by Symrise, for example.

The microcapsules also form an aqueous solution when the material of the microcapsules dissolves in water, the first functional component and surfactant becoming mixed in the aqueous solution, allowing production by reduced pressure drying while spraying the aqueous solution.

The solvent is one that is capable of holding the first functional component encapsulated in the microcapsules, and when the functional composition further includes a volatile second functional component dissolved in the solvent, the solvent preferably dissolves the second functional component and does not dissolve or swell the microcapsules.

The solvent used to disperse the microcapsules may be referred to as the "first solvent", in order to distinguish it from the second solvent that can be encapsulated in the microcapsules, as described below.

From the viewpoint of avoiding disintegration of the water-disintegratable microcapsules, the solvent is preferably a lipophilic solvent.

From the viewpoint of lipophilicity, the solvent has an IOB, described hereunder, of 0.00 or greater, and preferably no greater than 1.0, more preferably no greater than 0.8 and even more preferably no greater than 0.6.

The IOB (Inorganic Organic Balance) is an indicator of the hydrophilic-lipophilic balance, and as used herein, it is the value calculated by the following formula by Oda et al.:
IOB = Inorganic value/organic value.

The inorganic value and the organic value are based on the organic paradigm described in "Organic compound predictions and organic paradigms" by Fujita A., Kagaku no Ryoiki (Journal of Japanese Chemistry), Vol.11, No.10 (1957) p.719-725.

The organic values and inorganic values of major groups, according to Fujita, are summarized in Table 1 below.

**Table 1**

| Group | Inorganic value | Organic value |
|---|---|---|
| -COOH | 150 | 0 |
| -OH | 100 | 0 |
| -O-CO-O- | 80 | 0 |
| -CO- | 65 | 0 |
| -COOR | 60 | 0 |
| -O- | 20 | 0 |
| Triple bond | 3 | 0 |
| Double bond | 2 | 0 |
| CH₂ | 0 | 20 |
| *iso*-branch | 0 | -10 |
| *tert*-branch | 0 | -20 |
| Light metal (salt) | ≥500 | 0 |
| Heavy metal (salt), amine, NH₃ salt | ≥400 | 0 |

In addition, the solvent preferably has a kinematic viscosity of 0.01 to 80 mm²/s at 40°C, from the viewpoint of coatability of the functional composition.

The kinematic viscosity is measured according to JIS K 2283:2000, "5. Kinematic Viscosity Test Method", using a Cannon-Fenske reverse-flow viscometer, at a testing temperature of 40°C.

The solvent has a vapor pressure of preferably 0.00 to 0.01 Pa, more preferably 0.000 to 0.001 Pa and even more preferably 0.0000 to 0.0001 Pa at 1 atmosphere, 25°C. Considering that the absorbent article produced by the production method of the disclosure is to be used in contact with the human body, the solvent has a vapor pressure of preferably 0.00 to 0.01 Pa, more preferably 0.000 to 0.001 Pa and even more preferably 0.0000 to 0.0001 Pa at 1 atmosphere, 40°C. If the vapor pressure is high, gasification may occur during storage and the amount of solvent and second functional component as an optional component may be reduced, often creating problems, such as odor during wear.

Examples of solvents include lipophilic alcohol-based solvents, ester-based solvents, ether-based solvents, ketone-based solvents and hydrocarbon-based solvents.

An example of the hydrocarbon-based solvent is liquid paraffin, and an example of the ester-based solvent is isopropyl myristate.

The solvent may further include components having an IOB of 0.00 to 0.60, a kinematic viscosity of 0.01 to 80 mm²/s at 40°C, a water holding percentage of 0.01 to 4.0 mass% and a weight-average molecular weight of less than 1,000 (these may collectively be referred to as "body fluid lubricity imparting agent").

The body fluid lubricity imparting agent is the same component as the "blood modifying agent" described in International Patent Publication No. WO2012/133724, previously filed by the present applicant, and is likewise the same component as the "blood slipping agent" described in International Patent Publication No. WO2013/129236, also by the present applicant.

If the solvent includes a body fluid lubricity imparting agent, it will be possible for body fluid that has reached the liquid-permeable layer to rapidly slip down into the absorbent body over long periods of time.

Examples of body fluid lubricity-imparting materials include triglycerides, such as PANACET 810s and PANACET 800 by NOF Corp., and hydrocarbons, such as PARLEAM 6 by NOF Corp.

The solvent may include components selected from the group consisting of PPG-4 butyl ether, PPG-12 butyl ether, PPG-17 butyl ether, PPG-20 butyl ether, PPG-24 butyl ether, PPG-33 butyl ether, PPG-40 butyl ether, PPG-52 butyl ether, PPG-3 myristyl ether, PPG-10 cetyl ether, PPG-11 stearyl ether, PPG-15 stearyl ether, PPG-2 lanolin alcohol ether, PPG-5 lanolin alcohol ether, PPG-10 lanolin alcohol ether, PPG-20 lanolin alcohol ether, PPG-26 oleate, PPG-36 oleate, PPG-5.5 castorate, PPG-6 glyceryl ether, PPG-8 glyceryl ether, PPG-10 glyceryl ether, PPG-16 glyceryl ether, PPG-9 diglyceryl ether, PPG-14 diglyceryl ether, PPG-25 sorbitol and PPG-33 sorbitol, as well as any combination thereof.

The solvent may also include components selected from the group consisting of PPG-30 cetyl ether, PPG-15 isohexadecyl ether, PPG-4 lauryl ether, PPG-20 distearate, PPG-12 dilaurate, PPG-15 dicocoate, PPG-10 cetyl phosphate, PPG-9 laurate, PPG-8 dioctate, PPG-15 stearate, PPG-8 diethyl hexylate, PPG-10 glyceryl stearate, PPG-2 cocamide, PPG-10 tallow amine, PPG-10 oleamide, PPG-5 sucrose cocoate, PPG-20 methylglucose ether distearate, PPG-20 methylglucose ether acetate, PPG-20 sorbitan tristearate, PPG-20 methylglucose ether distearate, PPG-15 stearyl ether benzoate, PPG-10 sorbitan monostearate, PPG-10 hydrogenated castor oil, PPG-10 cetyl ether phosphate, PPG-10 dinonyl phenolate, PPG-7 lauryl ether, PPG-5 lanolin wax ether, PPG-5 lanolin wax, PPG-4 jojoba alcohol ether, PPG-3 myristyl ether propionate, PPG-3 benzyl ether myristate, PPG-3 hydrogenated castor oil, PPG-3-hydroxyethylsoyamide, PPG-2 lanolin alcohol ether and PPG-1 coconut fatty acid isopropanolamide, as well as any combination thereof.

In the production method of the disclosure, when the functional composition includes a second functional component dissolved in a solvent, the functional composition present in the functional composition-coated region of the produced absorbent article may exhibit a desired function at a desired timing. Specifically, it is as follows.

When the user unseals the absorbent article, the second functional component dissolved in the solvent (first solvent) volatilizes, and the function of the second functional component is exhibited for the user. For example, when the second functional component is an aromatic component, the user senses an ambient drifting aroma.

When the second functional component is a deodorant component, and the user unseals the absorbent article, the second functional component exhibits its deodorant function and the user is less likely to sense the odor from the fluid absorbed by an absorbent article that requires exchanging.

The amount of release of the second functional component can be varied by the amount of the second functional component and the vapor pressure of the second functional component.

For example, the second functional component can be released to the surrounding area at a high concentration in a short period of time by increasing the amount of the second functional component in the absorbent article, or by selecting a second functional component with a high vapor pressure.

By selecting a second functional component with a low vapor pressure, for example, on the other hand, the second functional component can be released into the surrounding area over a long period of time.

When the absorbent article is to be sold as a package of individually packaged absorbent articles, the form of the individual packages and the packaged form may be adjusted; for example, the absorbent articles may be individually packaged with air permeable nonwoven fabrics, and the multiple individually packaged absorbent articles packaged with a polymer film to allow the function of the second functional component to be exhibited after the user has opened the package.

When the absorbent article is then worn by the wearer, the body heat of the wearer promotes gasification of the second functional component so that the function of the second functional component is promoted. For example, when the second functional component is an aromatic component, cooling component, deodorant component, antibacterial component or skin care component, this will promote the aromatic function, cooling function, deodorant function, antibacterial function or skin care function, respectively.

When the absorbent article then absorbs fluid, the absorbed fluid disintegrates the water-disintegratable microcapsules, causing release of the first functional component from the microcapsules so that its function is exhibited. For example, when the first functional component is an aromatic component, cooling component, deodorant component, antibacterial component or skin care component, this will allow the aromatic function, cooling function, deodorant function, antibacterial function or skin care function, respectively, to be exhibited.

The amount of release of the first functional component may be varied depending on the amount of the first functional component, the vapor pressure of the first functional component, the solubility of the microcapsules in water, the thickness of the microcapsule layer, and the particle sizes of the microcapsules.

For example, the first functional component can be released into the surrounding area at high concentration in a short period of time, by increasing the amount of the first functional component in the absorbent article, by selecting a first functional component with a high vapor pressure, by selecting the material of the microcapsules to be one with high solubility in water, by reducing the thickness of the microcapsule layer, or by reducing the particle sizes of the microcapsules.

Also, the first functional component can be released in a sustained manner, for example, by decreasing the amount of the first functional component in the absorbent article, by selecting a first functional component with a low vapor pressure, by selecting the material of the microcapsules to be one with low solubility in water, by increasing the thickness of the microcapsule layer, or by increasing the particle sizes of the microcapsules.

From the viewpoint of controlling the release property of the first functional component, the microcapsules may further include a solvent in addition to the first functional component (this solvent will also be referred to hereunder as the "second solvent").

The second solvent may be any of the same ones mentioned for the first solvent.

The functional composition includes the first functional component-containing microcapsules in a proportion of preferably 0.1 to 60 mass%, more preferably 5 to 40 mass% and even more preferably 10 to 30 mass%. This is from the viewpoint of the coatability of the functional composition.

The amount of the first functional component in the functional composition will differ depending on the function of the first functional component, but generally speaking the functional composition includes the first functional component in a proportion of preferably 0.01 to 30 mass%, more preferably 0.05 to 20 mass% and even more preferably 1 to 15 mass%.

When the functional composition includes a second functional component, the amount of the second functional component to be included in the functional composition will differ depending on the function of the second functional component, but generally speaking the functional composition includes the second functional component in a proportion of preferably 0.01 to 20 mass%, more preferably 0.05 to 15 mass% and even more preferably 0.1 to 10 mass%.

The absorbing layer in the absorbent article produced by the production method of the disclosure may include a super absorbent material. For this embodiment, the super absorbent material holds the solvent composing the functional composition in its surrounding area and adsorbs it, and therefore the absorbed fluid easily contacts with the microcapsules and the microcapsules readily dissolve.

Examples of high-water-absorbing materials include starch-based, cellulosic and synthetic polymer-based high-water-absorbing materials. Examples of starch-based or cellulosic high-water-absorbing materials include starch-acrylic acid (acrylate) graft copolymer, saponified starch-acrylonitrile copolymer and crosslinked sodium carboxymethyl cellulose, and examples of synthetic polymer-based high-water-absorbing materials include polyacrylic acid salt-based, polysulfonic acid salt-based, maleic anhydride salt-based, polyacrylamide-based, polyvinyl alcohol-based, polyethylene oxide-based, polyaspartic acid salt-based, polyglutamic acid salt-based, polyalginic acid salt-based, starch-based and cellulosic high water-absorbent resins, among which polyacrylic acid salt-based (especially sodium polyacrylate-based) high water-absorbent resins are preferred.

When the functional composition includes the first functional component and second functional component, they may be selected from any desired considerations. For example, by selecting the first functional component from among cooling components and the second functional component from among aromatic components, the wearer will be provided with an aroma after the absorbent article has been opened, and the wearer will also be provided with a cool feel after absorption of body fluid. Moreover, since night-use absorbent articles have larger sizes they tend to be prone to stuffy when worn, but if a cooling component is used as the first functional component, the wearer will be less likely to experience discomfort.

By selecting the first functional component and second functional component from among different aromatic components, the functional component can be used as an indicator material that notifies the user, and particularly a mother or caregiver for the disposable diaper, that body fluid has been absorbed.

In addition, if the first functional component and second functional component are selected from among the same aromatic components, then their amounts can be changed to accentuate the aroma. For example, prior to absorption of fluid, a faint aroma from the second functional component will drift, and after the fluid has been absorbed, the same aroma, but stronger than before absorption of the fluid, will drift from the first functional component.

Moreover, if a deodorant component, or an aromatic component, and preferably an aromatic component with a powerful aroma, is used as the first functional component, it will be possible to use the functional component as an aroma masking material.

Examples for the absorbent article produced by the production method of the disclosure include sanitary napkins, panty liners, disposable diapers and urine-absorbing pads.

### Examples

The present disclosure will now be explained in fuller detail by an example, with the understanding that the disclosure is not meant to be limited to the example.

### [Example 1]

INCAP MENTHOL/IPM by Symrise was prepared as microcapsules for encapsulation of the first functional component. The INCAP MENTHOL/IPM contained a cooling component (menthol) as the first functional component, and the microcapsule material was material-modified starch.

A solvent containing an aromatic component as the second functional component was prepared. The solvent was IPM (isopropyl myristate).

The microcapsules and the solvent were mixed in a mass ratio of 50:50 to prepare functional composition No.1.

The production processes shown in Fig. 3 were employed to produce a sanitary napkin No.1 as shown in Fig. 1, and then sanitary napkin No.1 was individually packaged with a polyethylene film.

The basis weight of the functional composition in the functional composition-coated regions was 4 g/m².

When sanitary napkin No.1 was used by volunteer subjects, it was reported that the fragrant aroma from sanitary napkin No.1 drifted after opening of the individual package and during fitting, and that after sanitary napkin No.1 had absorbed menstrual blood, a cool sensation was felt in the region contacting with absorbent article No.1.

Specifically, the present disclosure relates to the following aspects J1 to J14.

### [J1]

A method of producing an absorbent article including a liquid-permeable layer, a liquid-impermeable layer and an absorbing layer between the liquid-permeable layer and liquid-impermeable layer, including the steps of:
forming a functional composition by mixing water-disintegratable microcapsules encapsulating a volatile functional component and a solvent capable of holding the functional component encapsulated in the microcapsules;
forming a functional composition-coated region on a coating surface of a layer to be coated by coating the functional composition onto at least a portion of the coating surface of the layer to be coated; and
stacking the liquid-permeable layer, the absorbing layer and the liquid-impermeable layer sandwiching an adhesive-coated region that is coated with an adhesive.

### [J2]

The method according to J1, wherein the layer to be coated has a fiber density of 0.02 to 0.1 g/cm³.

### [J3]

The method according to J1 or J2, wherein the layer to be coated has a thickness of 0.2 to 2.0 mm.

### [J4]

The method according to any one of J1 to J3, wherein in the step of forming the functional composition-coated region, the coating surface is coated with the functional composition by a contact coating method.

### [J5]

The method according to any one of J1 to J4, wherein the coating surface or a surface facing the coating surface has an adhesive-coated region, and in the step of forming the functional composition-coated region, at least a portion of the adhesive-coated region of the coating surface or a region facing the adhesive-coated region of the coating surface are coated with the functional composition, the adhesive-coated region having a microcapsule-holding region that holds the microcapsules, in the areas where they overlap with the functional composition-coated region in a thickness direction of the absorbent article.

### [J6]

The method according to any one of J1 to J4, the method further includes, after the step of forming the functional composition-coated region, a step of coating the adhesive onto at least a portion of the functional composition-coated region or a region facing the functional composition-coated region, forming an adhesive-coated region that overlaps with the functional composition-coated region in a thickness direction of the absorbent article.

### [J 7]

The method according to any one of J1 to J6, the method further includes, after the step of forming the functional composition-coated region, a step of forming an embossed section by embossing at least the liquid-permeable layer and the absorbing layer sandwiching the functional composition-coated region.

### [J8]

The method according to any one of J1 to J7, wherein the absorbing layer includes a super absorbent material.

### [J9]

The method according to any one of J1 to J8, wherein the functional component has a function selected from the group consisting of aromatic functions, cooling functions, deodorant functions, antibacterial functions, skin care functions, and any combination thereof.

### [J10]

The method according to any one of J1 to J9, wherein the functional composition further includes a volatile second functional component dissolved in the solvent.

### [J11]

The method according to any one of J1 to J9, the methods further includes, either before or after the step of forming the functional composition-coated region, a step of coating the coating surface with a solvent containing the second functional component.

### [J 12]

The method according to J10 or J11, wherein the second functional component has a function selected from the group consisting of aromatic functions, cooling functions, deodorant functions, antibacterial functions, skin care functions, and any combination thereof.

### [J13]

The method according to any one of J1 to J12, wherein the coating surface is a non-skin contact surface of the liquid-permeable layer.

### [J14]

The method according to any one of J1 to J12, wherein the absorbent article includes an auxiliary sheet layer between the liquid-permeable layer and the absorbing layer, and in the step of forming the functional composition-coated region, a surface on the liquid-permeable layer side or a surface on the absorbing layer side of the auxiliary sheet layer is coated with the functional composition, to form the functional composition-coated region on the surface on the liquid-permeable layer side or the surface on the absorbing layer side.

### Reference Signs List

- 1: Absorbent article
- 2: Top sheet
- 3: Back sheet
- 4: Absorbent body
- 5: Side sheet
- 6: Side flap
- 7: Embossed section
- 8: Functional composition-coated region
- 9: Adhesive-coated region
- 10: Adhered region
- 11: Microcapsule-holding region
- 12: Auxiliary sheet
- 13: Coater
- 101: Top sheet roll
- 102: Functional composition-coating device
- 103, 122: Adhesive coaters
- 104: Absorbent body production apparatus
- 105: Material feeder
- 106: Suction drum
- 107: Concave die
- 108: Suction section
- 109, 123: Stacked bodies
- 111, 131: Embossing rolls
- 121: Back sheet roll
- 141: Cutter

## Claims

1. A method of producing an absorbent article including a liquid-permeable layer, a liquid-impermeable layer and an absorbing layer between the liquid-permeable layer and liquid-impermeable layer, including the steps of:
forming a functional composition by mixing water-disintegratable microcapsules encapsulating a volatile functional component and a solvent capable of holding the functional component encapsulated in the microcapsules;
forming a functional composition-coated region on a coating surface of a layer to be coated by coating the functional composition onto at least a portion of the coating surface of the layer to be coated; and
stacking the liquid-permeable layer, the absorbing layer and the liquid-impermeable layer sandwiching an adhesive-coated region that is coated with an adhesive.

2. The method according to claim 1, wherein the layer to be coated has a fiber density of 0.02 to 0.1 g/cm³.

3. The method according to claim 1 or 2, wherein the layer to be coated has a thickness of 0.2 to 2.0 mm.

4. The method according to any one of claims 1 to 3, wherein in the step of forming the functional composition-coated region, the coating surface is coated with the functional composition by a contact coating method.

5. The method according to any one of claims 1 to 4, wherein the coating surface or a surface facing the coating surface has an adhesive-coated region, and in the step of forming the functional composition-coated region, at least a portion of the adhesive-coated region of the coating surface or a region facing the adhesive-coated region of the coating surface are coated with the functional composition, the adhesive-coated region having a microcapsule-holding region that holds the microcapsules, in the areas where they overlap with the functional composition-coated region in a thickness direction of the absorbent article.

6. The method according to any one of claims 1 to 4, the method further includes, after the step of forming the functional composition-coated region, a step of coating the adhesive onto at least a portion of the functional composition-coated region or a region facing the functional composition-coated region, forming an adhesive-coated region that overlaps with the functional composition-coated region in a thickness direction of the absorbent article.

7. The method according to any one of claims 1 to 6, a the method further includes, after the step of forming the functional composition-coated region, a step of forming an embossed section by embossing at least the liquid-permeable layer and the absorbing layer sandwiching the functional composition-coated region.

8. The method according to any one of claims 1 to 7, wherein the absorbing layer includes a super absorbent material.

9. The method according to any one of claims 1 to 8, wherein the functional component has a function selected from the group consisting of aromatic functions, cooling functions, deodorant functions, antibacterial functions, skin care functions, and any combination thereof.

10. The method according to any one of claims 1 to 9, wherein the functional composition further includes a volatile second functional component dissolved in the solvent.

11. The method according to any one of claims 1 to 9, the methods further includes, either before or after the step of forming the functional composition-coated region, a step of coating the coating surface with a solvent containing the second functional component.

12. The method according to claim 10 or 11, wherein the second functional component has a function selected from the group consisting of aromatic functions, cooling functions, deodorant functions, antibacterial functions, skin care functions, and any combination thereof.

13. The method according to any one of claims 1 to 12, wherein the coating surface is a non-skin contact surface of the liquid-permeable layer.

14. The method according to any one of claims 1 to 12, wherein the absorbent article includes an auxiliary sheet layer between the liquid-permeable layer and the absorbing layer, and in the step of forming the functional composition-coated region, a surface on the liquid-permeable layer side or a surface on the absorbing layer side of the auxiliary sheet layer is coated with the functional composition, to form the functional composition-coated region on the surface on the liquid-permeable layer side or the surface on the absorbing layer side.
